# DEMANDE DE BREVET EUROPEEN

(11) **EP 1 018 334 A1**
(43) Date de publication de la demande: **12.07.2000**
(21) Numéro de dépôt: 99402979.1
(22) Date de dépôt: 30.11.1999
(51) Int. Cl.: A61K 7/48

(54) **Composition cosmétique comprenant un copolymère styrène/acrylate et une phase grasse**

(30) Priorité: 06.01.1999 FR 9900055
(71) Demandeur: L'OREAL, 75008 Paris (FR)
(72) Inventeur: Collin, Nathalie, 92330 Sceaux (FR); Bodelin, Sophie, 92170 Vanves (FR)
(74) Mandataire: Kromer, Christophe

(57) **Abrégé**

L'invention a pour objet une composition à application topique comprenant, dans un support cosmétiquement, dermatologiquement, hygiéniquement ou pharmaceutiquement acceptable, au moins une phase grasse et au moins un polymère lipophile, caractérisée par le fait que le polymère lipophile est un copolymère de tertio-butyl styrène/ méthacrylate d'alkyle en C1-C10/ acrylate d'alkyle en C1-C10.

La composition permet d'obtenir un film de très bonne tenue et présentant de bonnes propriétés de non transfert. Le film obtenu est notamment bien résistant à l'eau, aux frottements par exemple des doigts ou des vêtements, à la transpiration et au sébum.

## Description

La présente invention a pour objet une composition contenant au moins un copolymère styrène/acrylate, destinée en particulier aux domaines cosmétique, dermatologique, pharmaceutique, et hygiénique. Plus précisément, l'invention se rapporte à une composition pour le soin ou le maquillage de la peau, y compris les lèvres, ou encore des phanères comme les cils, les sourcils, les cheveux et les ongles.

Cette composition peut se présenter sous forme de mascara, d'eye-liner, de produit pour les lèvres, de fard à joues ou à paupières, de fond de teint, de produit de maquillage du corps, de produit anti-cernes, de composition de protection solaire, de coloration de la peau, de produit de soin de la peau.

Les produits de maquillage ou de soin de la peau ou des lèvres d'êtres humains comme les eye-liners, les fonds de teints, les rouges à lèvres, ou bien encore des cils comme les mascaras, contiennent généralement des corps gras tels que des cires et des huiles, des pigments et/ou charges et, éventuellement des additifs comme des actifs cosmétiques ou dermatologiques. Ces compositions, lorsqu'elles sont appliquées sur la peau ou les cils, laissent un film sur ces derniers qui ne présente pas toujours une bonne résistance à l'eau, lors de baignade ou de douche par exemple, et/ou aux larmes et/ou à la transpiration et/ou au sébum et/ou aux frottements des doigts, des vêtements. Le film est ainsi fragilisé et le maquillage ne présente plus alors une bonne tenue dans le temps.

D'autre part, ces compositions présentent également l'inconvénient de transférer, c'est-à-dire de se déposer au moins en partie, en laissant des traces sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de maquillage. Par ailleurs, l'apparition de traces inacceptables, notamment sur les cols de chemisier, peut écarter certaines femmes de l'utilisation de ce type de maquillage.

La présente invention a donc pour but de proposer une composition ne présentant pas les inconvénients ci-dessus, et conduisant à la formation d'un film ayant une bonne tenue et résistant à l'eau.

La demanderesse a constaté, de façon tout à fait surprenante, que l'utilisation d'un copolymère de styrène/acrylate particulier, dans une composition cosmétique, dermatologique, pharmaceutique ou hygiénique, pouvait permettre d'obtenir un film de très bonne tenue et présentant de bonnes propriétés de non transfert. Le film obtenu est notamment bien résistant à l'eau, aux frottements par exemple des doigts ou des vêtements, à la transpiration et au sébum. Le film est également souple, flexible, brillant et non collant.

De façon plus précise, la présente invention a pour objet une composition à application topique comprenant, dans un support cosmétiquement, dermatologiquement, hygiéniquement ou pharmaceutiquement acceptable, au moins une phase grasse et au moins un polymère lipophile, caractérisée par le fait que le polymère lipophile est un copolymère de tertio-butyl styrène/ méthacrylate d'alkyle en C1-C10/ acrylate d'alkyle en C1-C10.

Un autre objet de l'invention est l'utilisation dans une composition cosmétique, dermatologique ou hygiénique, ou pour la fabrication d'une composition pharmaceutique, à application topique d'au moins un copolymère de tertio-butyl styrène/ méthacrylate d'alkyle en C1-C10/ acrylate d'alkyle en C1-C10, pour obtenir un film de bonne tenue et/ou résistant à l'eau et/ou résistant aux frottements et/ou résistant à la transpiration et/ou résistant au sébum et/ou ayant des propriétés de non transfert, la composition comprenant au moins une phase grasse.

L'invention a aussi pour objet un procédé cosmétique de maquillage ou de traitement non thérapeutique de la peau ou des phanères consistant à appliquer sur ceux-ci une composition telle que décrite précédemment.

On entend par polymère lipophile un polymère apte à être solubilisé et/ou dispersé dans la phase grasse de la composition.

De préférence, le polymère lipophile est un copolymère de tertio-butyl styrène/ méthacrylate d'alkyle en C1-C10/ acrylate d'alkyle en C1-C10 et d'un monomère aromatique vinylique différent du tertio-butyl styrène.

Avantageusement, le polymère lipophile peut être un copolymère issu de la polymérisation de :
a) 10 à 55 % en poids, du poids total de monomères, de para tertio-butyl styrène,
b) 20 à 80 % en poids de monomère méthacrylate d'alkyle en C1-C10,
c) 2 à 25 % en poids de monomère acrylate d'alkyle en C1-C10,
d) de 0 à 40 % en poids de monomère aromatique vinylique différent du para tertio-butyl styrène.

Le groupe alkyle du monomère méthacrylate d'alkyle en C1-C10 peut être linéaire ou ramifié et peut comporter de préférence de 1 à 4 atomes de carbone, et mieux 4 carbones. Comme méthacrylate d'alkyle en C1-C10, on peut par exemple citer le méthacrylate de méthyle, le méthacrylate d'éthyle, le méthacrylate de propyle, le méthacrylate d'isobutyle, le méthacrylate de n-butyle. Le monomère méthacrylate particulièrement préféré est le méthacrylate d'isobutyle.

Le groupe alkyle du monomère acrylate d'alkyle en C1-C10 peut être linéaire ou ramifié et peut comporter de préférence de 2 à 8 atomes de carbone, et mieux 8 atomes de carbone. Comme acrylate d'alkyle en C1-C10, on peut par exemple citer l'acrylate de propyle, l'acrylate d'isopropyle, l'acrylate de n-butyle, l'acrylate d'isobutyle, l'acrylate de pentyle, l'acrylate d'hexyle, l'acrylate de 2-éthyl hexyle. Le monomère acrylate particulièrement préféré est l'acrylate de 2-éthyl hexyle.

Le monomère aromatique vinylique éventuellement présent dans le copolymère peut être choisi parmi les monomères aromatiques vinyliques autres que le tertio-butyl styrène, connu pour polymériser par voir radicalaire. Comme monomère aromatique vinylique, on peut notamment utiliser ceux ayant de 8 à 20 atomes de carbone, et mieux ceux ayant de 4 à 14 atomes de carbone. De tels monomères sont par exemple le styrène, le 1-vinyl naphtalène, le 2-vinyl naphtalène, le 2-méthyl styrène (ou ortho-méthyl styrène), le 3-méthyl styrène, le 4-méthyl styrène (ou para-méthyl styrène), le 4-propyl styrène, le 4-cyclohexyl styrène, le 4-dodécyl styrène, le 2-éthyl 4-benzyl styrène, le 4-(phénylbutyl) styrène. On peut utiliser de préférence le styrène, le para-méthyl styrène, l'ortho méthyl styrène, et leurs mélanges. Le monomère aromatique vinylique particulièrement préféré est le para méthyl styrène.

Avantageusement, le copolymère peut être issu de la polymérisation de :
a) 10 à 30 % en poids de para tertio-butyl styrène,
b) 20 à 40 % en poids de para méthyl styrène,
c) 25 à 45 % en poids de méthacrylate d'isobutyle,
d) 8 à 25 % en poids d'acrylate de 2-éthyl hexyle.

Selon un mode particulier de l'invention, le copolymère peut être réticulé et donc comprendre, de façon connue, au moins un monomère éthylénique réticulant. Comme monomère réticulant, on peut notamment citer le diméthacrylate d'éthylène glycol, le diacrylate d'ethylène glycol, le divinyl benzène. On utilise de préférence le diméthacrylate d'éthylène glycol.
Le monomère réticulant peut être présent dans le copolymère en une teneur allant de 0,1 % à 10 % en poids, par rapport au poids total du mélange de monomères du copolymère.

Les copolymères de la composition selon l'invention sont connus et notamment décrits dans le brevet US-A- 5496905. De tels copolymères sont commercialisés sous les dénominations "PLIOWAY® ULTRA 200" et "PLIOWAY® ULTRA G 20" par la société GOODYEAR.

Ledit copolymère peut être présent dans la composition en une teneur allant de 0,5 % à 30 % en poids, par rapport au poids total de la composition, de préférence de 1 % à 20 % en poids, et mieux de 5 % à 15 % en poids.

La phase grasse de la composition selon l'invention permet d'incorporer facilement ledit copolymère de styrène/acrylate dans la composition.
La phase grasse peut comprendre au moins un corps gras qui peut être liquide, pâteux ou solide à température ambiante (25 °C en général). En particulier, le corps gras peut être choisi parmi les huiles, les cires, les corps gras pâteux, les gommes et leurs mélanges. Le corps gras peut être présent dans la composition en une teneur allant de 5 % à 99 % en poids, par rapport au poids total de la composition, de préférence de 10 % à 80 % en poids, et mieux de 20 % à 75 % en poids.
Avantageusement, la phase grasse de la composition comprend au moins un corps gras liquide à température ambiante.

Le corps gras liquide peut être une huile volatile. On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone. Les huiles hydrocarbonées volatiles préférées convenant pour la composition selon l'invention sont en particulier les huiles hydrocarbonées ayant de 8 à 16 atomes de carbones, et notamment les isoalcanes en C8-C16 (ou isoparaffines) et les esters ramifiés en C8-C16 comme l'isododécane (encore appelé 2,2,4,4,6-pentaméthylheptane), l'isodécane, l'isohexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. Les huiles volatiles hydrocarbonées sont particulièrement préférées car elles permettent une bonne dissolution du copolymère styrène/acrylate.

Comme huiles volatiles, on peut aussi utiliser les silicones volatiles, comme par exemple les huiles de silicones cycliques et volatiles, notamment celles ayant une viscosité ≤ 8 centistokes (8 10⁻⁶ m²/s), telles que l'octaméthylcyclotétrasiloxane, le décaméthylcyclopentasiloxane, l'hexadécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition selon l'invention en une teneur allant de 0 % à 80 % en poids (notamment de 1 % à 80 %), par rapport au poids total de la composition, de préférence de 0 % à 65 % en poids (notamment de 1 % à 65 %).

Le corps gras liquide peut également être choisi parmi les huiles non volatiles, et notamment les huiles hydrocarbonées et/ou siliconées et/ou fluorées non volatiles.
Comme huile hydrocarbonée non volatile, on peut notamment citer :
- les huiles hydrocarbonées d'origine animale telle que le perhydrosqualène ;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité ;
- les hydrocarbures linéaires ou ramifiés, d'origine minérale ou synthétique tels que la vaseline, les polydécènes, le polyisobutène hydrogéné tel que le parléam ;
- les esters et les éthers de synthèse comme les huiles de formule R₁COOR₂ dans laquelle R₁ représente le reste d'un acide gras supérieur comportant de 6 à 29 atomes de carbone et R₂ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisonanoate de diéthylène glycol et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique ou l'acide isostéarique ; et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 5 % en poids, par rapport au poids total de la composition, de préférence de 0 % à 2 % en poids, et mieux de 0,1 % à 2 % en poids.

Les cires peuvent être choisies parmi les cires d'origine animale, les cires d'origine végétale ou les cires d'origine synthétique.

Les cires susceptibles d'être utilisées dans la composition selon l'invention possèdent en règle générale un point de fusion compris entre 40 et 110 °C et ont une pénétrabilité à l'aiguille allant de 1 à 217. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Parmi les cires d'origine animale, on peut citer les cires d'abeille, les cires de lanoline et les cires d'insectes de Chine.
Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.
Parmi les cires d'origine minérale, on peut citer les paraffines, les cires microcristallines, les cires de Montan et les ozokérites.
Parmi les cires d'origine synthétique, on peut utiliser notamment les cires de polyoléfine et notamment les cires de polyéthylène, les cires obtenues par la synthèse de Fischer et Tropsch, les copolymères cireux ainsi que leurs esters, les cires de silicone.
Il est également possible d'utiliser des huiles d'origine animale ou végétale hydrogénées qui répondent toujours aux deux caractéristiques physiques mentionnées précédemment.
Parmi ces huiles, on peut citer les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, l'huile de jojoba hydrogénée, la lanoline hydrogénée et les huiles de palme hydrogénées.
Les cires utilisables selon la présente invention sont de préférence solides et rigides à température inférieure à 50 °C.

La composition selon l'invention peut comprendre de 0,1 % à 30 % en poids de cire, en poids par rapport au poids total de la composition, de préférence de 1 % à 25 % en poids.

De préférence, la composition selon l'invention peut comprendre :
- au moins une cire ayant une pénétrabilité à l'aiguille allant de 1 à 7, 5 (dite cire I), notamment en une teneur allant de 0,1 % à 20 % en poids par rapport au poids total de la composition, et
- au moins une cire ayant une pénétrabilité à l'aiguille supérieure à 7,5 et inférieure ou égale à 217 (dite cire II), notamment en une teneur allant de 0,1 % à 10 % en poids par rapport au poids total de la composition. Ce mélange de cire est notamment approprié lorsque la composition est destinée à être utilisée comme mascara ou eye-liner.

La composition peut également comprendre au moins une matière colorante comme les composés pulvérulents et/ou les colorants liposolubles, par exemple à raison de 0,01 à 50 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 25 % du poids total de la composition et mieux de 1 à 20 %. Lorsque la composition est sous forme de poudre, elle peut contenir jusqu'à 95 % en poids de composés pulvérulents.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

La composition peut comprendre, en outre, un ou plusieurs actifs cosmétiques, dermatologiques, hygiéniques ou pharmaceutiques, tels que les agents hydratants, les vitamines, les acides gras essentiels, les protéines, les céramides, les filtres solaires, les agents anti-radicaux libres, les agents anti-inflamatoires, les agents bronzants. Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée. Ces actifs peuvent être par exemple utilisés en une teneur allant de 0,001 % à 20 % en poids, par rapport au poids total de la composition.

La composition selon l'invention peut, de plus, comprendre selon le type d'application envisagée, les constituants classiquement utilisés dans les domaines considérés, qui sont présents en une quantité appropriée à la forme galénique souhaitée.

La composition peut contenir, en outre, tout additif usuellement utilisés dans de telles compositions tels que des épaississants, des parfums, des conservateurs, des tensioactifs, des polymères filmogènes lipophiles ou hydrophiles, solubilisés ou dispersés dans la composition, notamment dispersés dans un milieu aqueux ou un milieu huileux tel que décrite dans les documents EP-A-749747 et EP-A-749746.

Lorsque la phase grasse de la composition selon l'invention comprend au moins un corps gras liquide à température ambiante, la composition peut comprendre, en outre, un agent épaississant de la phase grasse. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.
On peut également employer des silices traitées, des gommes de guar alkylées liposolubles.

Selon un mode de réalisation de l'invention, la composition peut être avantageusement anhydre, et peut contenir moins de 10 % d'eau par rapport au poids total de la composition. Elle peut alors se présenter sous forme de gel huileux, de liquide huileux, de pâte, de produit coulé en stick ou bâton, ou en coupelle, ou bien encore sous forme de poudre.

Selon un autre mode de réalisation, la composition peut comprendre en outre au moins une phase aqueuse et se présenter alors sous forme d'émulsion eau-dans-huile, huile-dans-eau, cire-dans-eau, eau-dans-cire, de dispersion vésiculaire contenant des lipides ioniques et/ou non ioniques. La teneur en eau peut aller de 10 % à 95 % en poids par rapport au poids total de la composition.

Ces compositions à application topique peuvent constituer notamment une composition cosmétique, dermatologique, hygiénique ou pharmaceutique de protection, de soin ou de traitement de la peau, notamment pour le visage, pour le cou, pour les mains ou pour le corps (par exemple crème de soin, huile solaire, gel corporel), une composition de maquillage, de composition de protection solaire ou une composition de bronzage artificiel.

La composition de maquillage peut être notamment un mascara, un eye-liner, un produit pour les lèvres (rouge à lèvres), un vernis à ongles, un fard à paupières ou à joues, un produit anti-cernes, un fond de teint, un produit de maquillage du corps du type tatouage provisoire ou semi-permanent.

L'invention est illustrée plus en détail dans les exemples suivants.

### Exemple 1 :

On a préparé un mascara ayant la composition suivante :
- Cire d'abeille 2,6 g
- Cire de carnauba 1,3 g
- Cire de paraffine 7,3 g
- Copolymère PLIOWAY® ULTRA 200 de GOODYEAR 10 g
- Acide stéarique 5,24 g
- Triéthanolamine 2,16 g
- Amino-2 méthyl-2 propane diol-1,3 0,45 g
- Agents épaississants hydrosolubles 3,92 g
- Polymères filmogène hydroslubles 0,99 g
- Pigments 4 g
- Conservateurs qs
- Eau qsp 100 g

Le PLIOWAY® ULTRA 200 est un copolymère para tertio-butyl styrène/paraméthyl styrène/acrylate de 2-éthyl hexyle/méthacrylate d'isobutyle.

Il peut être remplacé par le PLIOWAY® ULTRA G 20 qui est un copolymère para tertio-butyl styrène/para-méthyl styrène/acrylate de 2-éthyl hexyle/méthacrylate d'isobutyle/diméthacrylate d'éthylène glycol.

Le mascara s'applique facilement sur les cils et permet d'obtenir un maquillage présentant une bonne tenue supérieure à un jour, résistant à l'eau.

### Exemple 2 :

On a préparé un eye-liner ayant la composition suivante :
- Cires 6,9 g
- Pigments 10 g
- Agent épaississant 7 g
- Copolymère PLIOWAY® ULTRA 200 10 g
- Copolymère acétate de vinyle/stéarate d'allyle (65/35) (polymère liposoluble) 6 g
- Amidon de riz 0,95 g
- Hydrocarbures paraffiniques et naphténiques légers (Shell Solt de SHELL) qsp 100 g

On obtient un eye-liner waterproof qui s'applique facilement sur le bord des paupières et laisse, après l'application, un film homogène présentant une bonne tenue dans le temps à l'eau et à la transpiration. Le film ne s'altère pas pendant la journée et ne transfère pas.

### Exemple 3 :

On a préparé un mascara ayant la composition suivante :
- Cire de paraffine 2 g
- Cire de carnauba 4,2 g
- Cire d'abeille 7,4 g
- Polylaurate de vinyle (Mexomère PP de CHIMEX) 0,66 g
- Copolymère acétate de vinyle/stéarate d'allyle (65/35) 1,96 g
- Copolymère PLIOWAY® ULTRA G 20 de GOODYEAR 10 g
- Polymère en dispersion aqueuse* 2,5 g MA
- Amidon de riz 0,74 g
- Pigments 5 g
- Conservateurs qs
- Isododécane qs p 100 g

*Copolymère acétate de vinyle/acide crotonique/tert-butyl-4-benzoate de vinyle (65/10/25) neutralisé à 65 % par l'amino-2 méthyl-2 propanol-1 et plastifié à 25 % par l'adipate de diisopropyle, préparé selon l'enseignement du document EP-A-655234.

Le mascara s'applique facilement sur les cils et présente une bonne tenue tout au long de la journée. Le maquillage obtenu est résistant à l'eau et ne transfère pas.

## Revendications

1. Composition à application topique comprenant, dans un support cosmétiquement, dermatologiquement, hygiéniquement ou pharmaceutiquement acceptable, au moins une phase grasse et au moins un polymère lipophile, caractérisée par le fait que le polymère lipophile est un copolymère de tertio-butyl styrène/ méthacrylate d'alkyle en C1-C10/ acrylate d'alkyle en C1-C10.

2. Composition selon la revendication 1, caractérisée par le fait que le polymère lipophile est un copolymère de tertio-butyl styrène/ méthacrylate d'alkyle en C1- C10/ acrylate d'alkyle en C1-C10 et d'un monomère aromatique vinylique autre que le tertio-butyl styrène.

3. Composition selon la revendication 1 ou 2, caractérisée par le fait que le polymère lipophile est un copolymère issu de la polymérisation de :
a) 10 à 55 % en poids, du poids total de monomères, de para tertio-butyl styrène,
b) 20 à 80 % en poids de monomère méthacrylate d'alkyle en C1-C10,
c) 2 à 25 % en poids de monomère acrylate d'alkyle en C1-C10,
d) de 0 à 40 % en poids de monomère aromatique vinylique différent du para terio-butyl styrène.

4. Composition selon l'une quelconque des revendications précédentes, caractérisée par le fait que le radical alkyle du méthacrylate d'alkyle comporte de 1 à 4 atomes de carbone, et mieux 4 carbones.

5. Composition selon l'une quelconque des revendications précédentes, caractérisée par le fait que le méthacrylate d'alkyle est le méthacrylate d'isobutyle.

6. Composition selon l'une quelconque des revendications précédentes, caractérisée par le fait que le radical alkyle de l'acrylate d'alkyle comporte de 2 à 8 atomes de carbone, et mieux 8 atomes de carbone.

7. Composition selon l'une quelconque des revendications précédentes, caractérisée par le fait que l'acrylate d'alkyle est l'acrylate de 2-éthyl hexyle.

8. Composition selon l'une quelconque des revendications 2 à 7, caractérisée par le fait que le monomère aromatique vinylique contient de 4 à 14 atomes de carbone.

9. Composition selon l'une quelconque des revendications 2 à 8, caractérisée par le fait que le monomère aromatique vinylique est choisi dans le groupe formé par le styrène, le para méthyl styrène, l'ortho méthyl styrène, et leurs mélanges.

10. Composition selon l'une quelconque des revendications 2 à 9, caractérisée par le fait que le monomère aromatique vinylique est le para méthyl styrène.

11. Composition selon l'une quelconque des revendications précédentes, caractérisée par le fait que le polymère lipophile comprend de :
a) 10 à 30 % en poids de para tertio-butyl styrène,
b) 20 à 40 % en poids de para méthyl styrène,
c) 25 à 45 % en poids de méthacrylate d'isobutyle,
d) 8 à 25 % en poids d'acrylate de 2-éthyl hexyle.

12. Composition cosmétique selon l'une quelconque des revendications précédentes, caractérisée par le fait que le polymère lipophile comprend de :
a) 15 à 25 % en poids de para tertio-butyl styrène,
b) 25 à 35 % en poids de para méthyl styrène,
c) 30 à 40 % en poids de méthacrylate d'isobutyle,
d) 10 à 20 % en poids d'acrylate de 2-éthyl hexyle.

13. Composition selon l'une quelconque des revendications précédentes, caractérisée par le fait que copolymère comprend en outre au moins un monomère éthylénique réticulant.

14. Composition selon la revendication 13, caractérisée par le fait que le monomère réticulant est le diméthacrylate d'éthylène glycol.

15. Composition selon l'une quelconque des revendications précédentes, caractérisée par le fait que ledit copolymère est présent en une teneur allant de 0,5 % à 30 % en poids, par rapport au poids total de la composition, de préférence de 1 à 30 % en poids, et mieux de 5 % à 15 % en poids.

16. Composition selon l'une quelconque des revendications précédentes, caractérisée par le fait que la phase grasse contient au moins un corps gras choisis parmi les huiles, les cires, les gommes, les corps gras pâteux, d'origine animale, végétale, minérale ou de synthèse.

17. Composition cosmétique selon l'une quelconque des revendications précédentes, caractérisée par le fait que la phase grasse comprend au moins un corps gras présent en une teneur allant de 5 % à 99 % en poids, par rapport au poids total de la composition, de préférence de 10 % à 80 % en poids, et mieux de 20 % à 75 % en poids.

18. Composition selon l'une quelconque des revendications précédentes, caractérisée par le fait que la phase grasse comprend au moins une huile volatile.

19. Composition cosmétique selon l'une quelconque des revendications précédentes, caractérisée par le fait que la phase grasse comprend au moins une huile hydrocarbonée volatile.

20. Composition selon l'une quelconque des revendications précédentes, caractérisée par le fait que la phase grasse comprend au moins une huile hydrocarbonée volatile choisie dans le groupe formé par les isoalcanes en C8-C16 et les esters ramifiés en C8-C16.

21. Composition selon l'une quelconque des revendications précédentes, caractérisée par le fait que la phase grasse comprend au moins une cire.

22. Composition selon l'une quelconque des revendications précédentes, caractérisée par le fait qu'elle comprend au moins une matière colorante.

23. Composition selon la revendication 22, caractérisée par le fait que la matière colorante comprend au moins un composé pulvérulent choisi dans le groupe formé par les charges, les pigments, les nacres et leurs mélanges.

24. Composition selon l'une quelconque des revendications précédentes, caractérisée par le fait qu'elle comprend, en outre, au moins un actif choisi dans le groupe formé par les actifs cosmétiques, dermatologiques, hygiéniques ou pharmaceutiques.

25. Composition selon l'une quelconque des revendications précédentes, caractérisée par le fait qu'elle comprend, en outre , au moins un additif choisi dans le groupe formé par les épaississants, les parfums, les conservateurs, les tensioactifs, les polymère filmogène lipophiles ou hydrophiles solubilisés ou dispersés dans la composition.

26. Composition selon l'une quelconque des revendications précédentes, caractérisée par le fait qu'elle se présente sous forme de stick ou bâton, de coupelle, d'une pâte souple, de viscosité dynamique à 25 °C de l'ordre de 1 à 40 Pa.s., de gel huileux, de liquide huileux, de poudre.

27. Composition cosmétique selon l'une quelconque des revendications précédentes, caractérisée par le fait que la composition est anhydre.

28. Composition selon l'une quelconque des revendications précédentes, caractérisée par le fait qu'elle se présente sous forme d'une émulsion eau-dans-huile, huile-dans-eau, de dispersion vésiculaire contenant des lipides ioniques et/ou non ioniques.

29. Composition cosmétique selon l'une quelconque des revendications précédentes, caractérisée par le fait qu'elle se présente sous la forme d'un produit de soin et/ou de maquillage de la peau et/ou des phanères.

30. Composition cosmétique selon l'une quelconque des revendications précédentes, caractérisée par le fait qu'elle se présente sous la forme d'un mascara, d'un eye-liner, d'un fard à joue ou à paupières, d'un produit pour les lèvres, d'un produit anti-cernes, d'un maquillage du corps, de produit de protection, de soin ou de traitement de la peau, de composition de protection solaire, de composition de bronzage artificiel.

31. Utilisation dans ou pour la fabrication d'une composition à application topique d'au moins un copolymère de tertio-butyl styrène/ méthacrylate d'alkyle en C1-C10/ acrylate d'alkyle en C1-C10, pour obtenir un film de bonne tenue et/ou résistant à l'eau et/ou résistant aux frottements et/ou résistant à la transpiration et/ou résistant au sébum et/ou ayant des propriétés de non transfert, la composition comprenant au moins une phase grasse.

32. Utilisation selon la revendication 31, caractérisée par le fait que le polymère lipophile est un copolymère de tertio-butyl styrène/ méthacrylate d'alkyle en C1-C10/ acrylate d'alkyle en C1-C10 et d'un monomère aromatique vinylique différent du tertio-butyl styrène.

33. Utilisation selon la revendication 31 ou 32 ,caractérisée par le fait que le polymère lipophile comprend de :
a) 10 à 55 % en poids, du poids total de monomères, de para tertio-butyl styrène,
b) 20 à 80 % en poids de monomère méthacrylate d'alkyle en C1-C10,
c) 2 à 25 % en poids de monomère acrylate d'alkyle en C1-C10,
d) de 0 à 40 % en poids de monomère aromatique vinylique différent du para terio-butyl styrène.

34. Utilisation selon l'une quelconque des revendications 31 à 33, caractérisée par le fait que le groupe alkyle du méthacrylate d'alkyle comporte de 1 à 4 atomes de carbone, et mieux 4 carbones.

35. Utilisation selon l'une quelconque des revendications 31 à 34, caractérisée par le fait que le méthacrylate d'alkyle est le méthacrylate d'isobutyle.

36. Utilisation selon l'une quelconque des revendications 31 à 35, caractérisée par le fait que le groupe alkyle de l'acrylate d'alkyle comporte de 2 à 8 atomes de carbone, et mieux 8 atomes de carbone.

37. Utilisation selon l'une quelconque des revendications 31 à 36, caractérisée par le fait que l'acrylate d'alkyle est l'acrylate de 2-éthyl hexyle.

38. Utilisation selon l'une quelconque des revendications 32 à 37, caractérisée par le fait que le monomère aromatique vinylique contient de 4 à 14 atomes de carbone.

39. Utilisation selon l'une quelconque des revendications 32 à 38, caractérisée par le fait que le monomère aromatique vinylique est choisi dans le groupe formé par le styrène, le para méthyl styrène, l'ortho méthyl styrène, et leurs mélanges.

40. Utilisation selon l'une quelconque des revendications 32 à 39, caractérisée par le fait que le monomère aromatique vinylique est le para méthyl styrène.

41. Utilisation selon l'une quelconque des revendications 31 à 40, caractérisée par le fait que le polymère lipophile comprend de :
a) 10 à 30 % en poids de para tertio-butyl styrène,
b) 20 à 40 % en poids de para méthyl styrène,
c) 25 à 45 % en poids de méthacrylate d'isobutyle,
d) 8 à 25 % en poids d'acrylate de 2-éthyl hexyle.

42. Utilisation selon l'une quelconque des revendications 31 à 41, caractérisée par le fait que le polymère lipophile comprend de :
a) 15 à 25 % en poids de para tertio-butyl styrène,
b) 25 à 35 % en poids de para méthyl styrène,
c) 30 à 40 % en poids de méthacrylate d'isobutyle,
d) 10 à 20 % en poids d'acrylate de 2-éthyl hexyle.

43. Utilisation selon l'une quelconque des revendications 31 à 42, caractérisée par le fait que le polymère lipophile comprend en outre au moins un monomère éthylénique réticulant.

44. Utilisation selon la revendication 43, caractérisée par le fait que le monomère réticulant est le diméthacrylate d'éthylène glycol.

45. Procédé cosmétique de maquillage de la peau et/ou des phanères, caractérisé par le fait que l'on applique sur la peau et/ou les phanères une composition selon l'une quelconque des revendications 1 à 30.

46. Procédé de traitement non thérapeutique de la peau et/ou des phanères, caractérisé par le fait que l'on applique sur la peau et/ou les phanères une composition selon l'une quelconque des revendications 1 à 30.
